# DEMANDE DE BREVET EUROPEEN

(11) **EP 4 559 434 A1**
(43) Date de publication de la demande: **28.05.2025**
(21) Numéro de dépôt: 24214010.1
(22) Date de dépôt: 19.11.2024
(51) Int. Cl.: A61F 2/04, A61L 27/36

(54) **GREFFON UTILISABLE POUR LA RECONSTRUCTION URÉTRALE COMPRENANT UNE VEINE OMBILICALE**

(30) Priorité: 21.11.2023 FR 2312837
(71) Demandeur: TBF Genie Tissulaire (TBF), 69780 Mions (FR)
(72) Inventeur: BARNOUIN, Laurence, 38460 Venerieu (FR)
(74) Mandataire: Tripoz, Inès

(57) **Abrégé**

La présente invention concerne un greffon utilisable pour la reconstruction urétrale présentant une forme partiellement tubulaire non collabable, ledit greffon comprenant au moins une veine ombilicale ou un segment de veine ombilicale disposée de telle sorte que l'épithélium vasculaire est orienté vers la face intérieure de la courbure.

## Description

La présente invention concerne le domaine des greffons utilisables pour la reconstruction urétrale.

La sténose urétrale se caractérise par un rétrécissement de l'urètre objectivé chez l'Homme, par un débit mictionnel inférieur à 16 mL/s. De nos jours, les sténoses urétrales sont principalement dues à des causes iatrogènes ou traumatiques (Guy Le Toux, et al., Med Buccale Chir Buccale 23 (1) 45-50 (2017)).

En première intention, le traitement de la sténose consiste soit en une dilatation du segment pathologique, soit en une incision endoscopique ou urétrotomie. La chirurgie résectrice de la sténose associée à une greffe vient en deuxième intention. C'est d'abord la muqueuse vésicale qui a été utilisée comme greffon, puis la peau du cuir chevelu, du pénis ou du scrotum.

La muqueuse buccale est employée comme greffon pour la première fois en 1941, mais il faut attendre 2002 pour voir publier les premières études multicentriques sur cette technique. Elle est à présent devenue le traitement de référence.

L'emploi de la muqueuse buccale dans le cadre d'une urétroplastie nécessite un prélèvement sur le patient ne pouvant s'effectuer que dans une quantité limitée sans provoquer de gêne trop importante pour le donneur. Un des inconvénients de cette méthode est donc la difficulté de traiter des sténoses étendues.

En particulier, les sténoses longues et/ou multi-segmentaires sont particulièrement complexes à traiter chirurgicalement, avec des urétroplasties alors plus complexes, plus longues avec parfois plusieurs temps opératoires, avec plus de complications et de risques d'échec (F.-X. Madec, et al., Prog Urol, 2021, 16, 31, 1055-1071).

D'autre part, les techniques actuelles d'urétroplastie par greffons tubulaires présentent un grand risque de resténose et sont souvent non recommandées.

Il existe donc un besoin de disposer de greffons permettant de traiter des sténoses étendues sans provoquer de gêne trop importante pour le patient, pouvant notamment se substituer à l'emploi de muqueuse buccale en tant que greffon.

De manière particulièrement inattendue, la demanderesse a mis au point un greffon utilisable pour la reconstruction urétrale présentant une forme tubulaire comprenant une lumière dont la section est circulaire ou sensiblement circulaire non collabable, ou une forme partiellement tubulaire, ledit greffon comprenant au moins une veine ombilicale ou un segment de veine ombilicale disposé de telle sorte que l'épithélium vasculaire est orienté vers la lumière lorsqu'il présente une forme tubulaire ou vers la face intérieure de la courbure lorsqu'il présente une forme sensiblement tubulaire, permettant de remédier aux désavantages des techniques connues ci-dessus décrites.

En raison de la longueur des veines ombilicales, le greffon selon la présente invention présente également l'avantage de permettre le traitement de sténoses urétrales étendues permettant ainsi de diminuer le nombre et le temps des interventions chirurgicales, les complications associées et les risques de récidives.

Il permet également d'éviter toute complication liée à l'étape de prélèvement de la muqueuse buccale telle que les fibroses, contractures labiales, lésions de nerfs, troubles de la sensibilité et/ou difficultés d'élocution.

Il présente également l'avantage d'être utilisé dans un champ plus large d'applications nécessitant une reconstruction urétrale, notamment pour le traitement chirurgical des hypospadias ou dans le cadre de métaidoïoplastie.

La demande FR3058321A1 déposée au nom de la demanderesse divulgue un guide de nerfs implantable non collabable constitué d'un segment d'artère ou de veine ombilicale dont la paroi est retournée, c'est-à-dire dont la gelée de Wharton est située au centre de la lumière du guide, caractérisé en ce qu'il est viro inactivé et lyophilisé. Cette demande ne divulgue ni ne suggère l'utilisation d'un tel guide pour remplacer l'urètre pathologique.

Le greffon mis au point par la demanderesse selon la présente invention présente une forme tubulaire constitué d'un segment de veine ombilicale qui est selon la présente invention disposée de sorte que l'épithélium vasculaire est orienté vers la lumière de la forme tubulaire, c'est-à-dire non retournée.

De manière tout à fait surprenante, la présence de l'épithélium vasculaire à l'intérieur de la forme tubulaire confère au greffon selon la présente invention la capacité à permettre la régénération de l'urètre pathologique en facilitant la prolifération de l'épithélium urétral.

Ainsi, le greffon selon la présente invention présente, de manière tout à fait inattendue, une capacité de régénération de l'urètre au moins équivalente à celle obtenue à partir d'une greffe autologue d'urètre.

La demande FR3110384A1 déposée au nom de la demanderesse divulgue une bandelette sous urétrale comprenant un vaisseau ombilical ou un fragment de celui-ci aplati suite à l'étape de lyophilisation. Cette demande ne divulgue ni ne suggère l'utilisation de telles bandelettes pour remplacer l'urètre pathologique puisque ces structures sont plates et ne disposent donc pas de lumière interne, elles ne permettent pas d'être implantées en lieu et place de la portion de l'urètre pathologique sans que le débit mictionnel ne soit altéré.

Par sa forme tubulaire, le greffon selon la présente invention possède de manière tout à fait inattendue la capacité à être implanté en lieu et place de la portion pathologique de l'urètre à remplacer sans que le débit mictionnel ne soit altéré.

De manière inattendue, la demanderesse est également parvenue à conserver la nature non collabable à l'inverse de la demande FR3110384A1 et malgré l'absence de retournement du vaisseau par rapport à la demande FR3058321A1.

La demande US 3,988,782 A est relative à l'utilisation d'artères ou de veines ombilicales flexibles et durcies pour la fabrication de prothèses. Les prothèses selon cette demande sont notamment décrites comme suffisamment souples pour pouvoir être fendues et étalées à plat, elles ne sont donc pas partiellement tubulaires. Les artères ou les veines ombilicales décrites dans cette demande sont durcies au moyen d'une solution d'aldéhyde, en particulier de glutaraldéhyde. Le durcissement est donc obtenu par réticulation des protéines des artères ou les veines ombilicales. Le brevet US 7,294,144 B1 divulgue en outre que l'effet du glutaraldéhyde employé dans la demande US 3,988,782 A est de relativement courte durée.

La demande FR2410997 (A1) divulgue des prothèses tubulaires obtenues à partir de cordon ombilical réticulé et siliconé. Le procédé de fabrication de ces prothèses comprend également le traitement par une solution d'aldéhyde et comprend en outre une étape de bain dans une solution de silicone. Il ne divulgue en outre pas de formes partiellement tubulaires.

Le greffon de la présente invention diffère des produits décrits dans ces demandes en ce que la veine ombilicale est durcie sans réticulation des protéines. Il présente donc l'avantage de conserver la structure native des protéines de la veine ombilicale et donc une meilleure biocompatibilité, et sa capacité d'intégration en particulier pour la vascularisation et/ou la cicatrisation de l'urètre, et/ou la capacité à permettre la colonisation de l'épithélium urétral en seront améliorées. De plus, son procédé de fabrication n'utilise pas de solution d'aldéhyde, un composé hautement toxique. Il permet en outre un maintien de la forme partiellement tubulaire ou tubulaire pendant une durée plus longue que celle obtenue selon la demande US 3,988,782 A1.

Le brevet US 7,294,144 B1 divulgue des greffons vasculaires comprenant un vaisseau ombilical lyophilisé puis réhydraté avant implantation. Un stent en nylon est placé à l'intérieur du vaisseau ombilical de manière à maintenir la séparation des parois du vaisseau. Le vaisseau selon ce brevet n'est donc pas non collabable. Il ne divulgue pas non plus de greffons utilisables pour la reconstruction urétrale ni de formes partiellement tubulaires.

En comparaison, le greffon selon la présente invention ne nécessite pas de dispositif externe permettant d'éviter le collapsus de la forme tubulaire ou partiellement tubulaire.

La présente invention a pour premier objet un greffon utilisable pour la reconstruction urétrale présentant une forme tubulaire comprenant une lumière dont la section est circulaire ou sensiblement circulaire non collabable, ou présentant une forme partiellement tubulaire non collabable, ledit greffon comprenant au moins une veine ombilicale ou un segment de veine ombilicale disposée de telle sorte que l'épithélium vasculaire est orienté vers la lumière lorsqu'il présente une forme tubulaire ou vers la face intérieure de la courbure lorsqu'il présente une forme partiellement tubulaire.

Au sens de la présente invention, on entend par « greffon » tout ou partie, ou assemblage de parties d'un tissu, d'un organe ou d'une partie d'organe prélevé sur un donneur et destiné à être transplanté dans un organisme receveur.

L'ensemble des modes de réalisation ci-après décrits sont combinables dans la mesure où lesdits modes ne sont pas incompatibles.

Dans un mode de réalisation, le greffon selon la présente invention est caractérisé en ce qu'il est d'origine humaine ou animale.

Dans un mode de réalisation, le greffon selon la présente invention est caractérisé en ce qu'il est d'origine humaine.

Tout matériel biologique humain employé selon la présente invention, en particulier la veine ombilicale, est issu de donneurs consentants en accord avec les exigences des directives Européennes et la Loi Bioéthique. Par exigence sanitaire relative aux dons de tissus et de cellules d'origine humaine, une qualification socio-clinique du donneur et biologique en amont du donneur est systématique. Cette qualification implique une recherche de virus HIV, hépatites B, C, HTLV et de la bactérie tréponème pâle responsable de la syphilis.

Dans un mode de réalisation, le greffon selon la présente invention est caractérisé en ce qu'il mesure de 1 à 50 cm de longueur.

Dans un mode de réalisation, le greffon selon la présente invention est caractérisé en ce qu'il mesure de 2 à 40 cm de longueur.

Dans un mode de réalisation, le greffon selon la présente invention est caractérisé en ce qu'il mesure de 3 à 30 cm de longueur.

Dans un mode de réalisation, le greffon selon la présente invention est caractérisé en ce qu'il mesure de 4 à 20 cm de longueur.

Dans un mode de réalisation, le greffon selon l'invention est caractérisé en ce qu'il est stérile.

Selon la présente invention, on entend par « stérile » une structure exempte de germe, à l'état naturel ou après stérilisation.

La stérilisation pourra être effectuée par toute les méthodes classiquement utilisées par l'Homme du métier.

Dans un mode de réalisation, le greffon selon l'invention est caractérisé en ce qu'il est stérilisé par irradiation gamma.

Dans un mode de réalisation, le greffon selon l'invention est caractérisé en ce qu'il est viro-inactivée.

Par « viro-inactivation », on entend une technique qui permet de réduire fortement ou totalement, et définitivement, la capacité d'action des virus. Ceux-ci, définis comme inactivés, perdent leurs capacités pathogéniques et de réplication par une diminution de leur population de 4 log lors des titrages résiduels qui suivent une ou deux étapes chimiques indépendantes, que ce soit sur des virus enveloppés, ou non enveloppés, ADN ou ARN.

Un tel procédé est par exemple décrit dans le document WO2017/140914 au nom de TBF GENIE TISSULAIRE.

Dans un mode de réalisation, le greffon selon l'invention est caractérisé en ce qu'il est viro-inactivé selon les deux étapes de viro-inactivation chimiques du procédé décrit dans WO2017/140914.

Dans un mode de réalisation, le greffon selon l'invention est caractérisé en ce qu'il est viro-inactivé selon le procédé tel que décrit ci-après.

Le procédé de viro-inactivation mentionné ci-dessus permet en outre de conserver la tenue de la forme au cours du temps et de conserver le caractère non collabable du greffon.

Dans un mode de réalisation, le greffon selon l'invention est caractérisé en ce qu'il est lyophilisé.

La lyophilisation permet en outre d'augmenter la tenue de la forme au cours du temps et d'augmenter le caractère non collabable du greffon.

Au sens de la présente invention, on entend par « lyophilisation », une technique visant à dessécher un produit préalablement surgelé par sublimation. Plus précisément, le liquide à ôter du produit est dans un premier temps transformé en glace par congélation ; puis par une dessiccation primaire, sous vide, la glace est sublimée ; enfin par une dessiccation secondaire, les molécules d'eau à la surface du produit sont extraites par désorption.

Dans un mode de réalisation, le greffon selon l'invention est caractérisé en ce qu'il est exempt de membrane de cordon ombilical.

Dans un mode de réalisation, le greffon selon l'invention est caractérisé en ce qu'il n'a pas subi de traitement par une solution aqueuse d'aldéhyde.

On entend par aldéhyde notamment les aldéhydes cités dans la demande US3988782, à savoir notamment le glutaraldéhyde, l'amidon dialdéhyde, le formaldéhyde ou le glyoxal.

On entend par solution aqueuse d'aldéhyde une solution comprenant de 0,1 à 3,0 % en masse d'aldéhyde.

Dans un mode de réalisation, le greffon selon l'invention est caractérisé en ce qu'il n'a pas subi de traitement par une solution de glutaraldéhyde.

Dans un mode de réalisation, le greffon selon l'invention est caractérisé en que ses protéines ne sont pas réticulées.

Dans un mode de réalisation, le greffon selon l'invention est caractérisé en ce qu'il n'est pas siliconé.

On entend par greffon siliconé, un greffon ayant subi un traitement par un bain de silicone en solution à 4 à 5 % massique dans un solvant organique comme le toluène.

Dans un mode de réalisation, le greffon selon l'invention est caractérisé en ce qu'il est exempt de membrane amniotique.

Dans un mode de réalisation, le greffon selon l'invention est caractérisé en ce qu'il présente une forme tubulaire comprenant une lumière dont la section est circulaire ou sensiblement circulaire non collabable, ledit greffon comprenant au moins une veine ombilicale ou un segment de veine ombilicale disposé de telle sorte que l'épithélium vasculaire est orienté vers la lumière.

Au sens de la présente invention, on entend par « tubulaire » une forme à section circulaire ou sensiblement circulaire, de conduit, de tube ou de cylindre.

Au sens de la présente invention, on entend par « lumière » l'espace libre à l'intérieur de la forme tubulaire.

Au sens de la présente invention, on entend par « sensiblement circulaire » une forme légèrement ovoïde ou de cercle légèrement aplati. La forme sensiblement circulaire permet de conserver un diamètre minimal en tout point de la lumière suffisant pour permettre un débit mictionnel physiologique. Lorsque la forme est sensiblement circulaire, elle sera définie par un diamètre équivalent.

Dans un mode de réalisation, la lumière est caractérisée en ce qu'elle présente un diamètre minimal ou un diamètre équivalent minimal de 5 mm.

Dans un mode de réalisation, la lumière est caractérisée en ce qu'elle présente un diamètre minimal ou un diamètre équivalent minimal de 7 mm.

Dans un mode de réalisation, la lumière est caractérisée en ce qu'elle présente un diamètre minimal ou un diamètre équivalent minimal de 10 mm.

Dans un mode de réalisation, la lumière est caractérisée en ce qu'elle présente un diamètre ou un diamètre équivalent compris dans un intervalle allant de 2 à 15 mm.

Dans un mode de réalisation, la lumière est caractérisée en ce qu'elle présente un diamètre ou un diamètre équivalent compris dans un intervalle allant de 8 à 12 mm.

Dans un mode de réalisation, la lumière est caractérisée en ce qu'elle présente un diamètre ou un diamètre équivalent compris dans un intervalle allant de 2 à 7 mm.

Dans un mode de réalisation, le greffon présentant une forme tubulaire comprenant une lumière dont la section est circulaire ou sensiblement circulaire non collabable est caractérisé en ce qu'il présente un diamètre externe ou un diamètre équivalent externe compris dans un intervalle allant de 5 à 25 mm.

Dans un mode de réalisation, le greffon présentant une forme tubulaire comprenant une lumière dont la section est circulaire ou sensiblement circulaire non collabable est caractérisé en ce qu'il présente un diamètre externe ou un diamètre équivalent externe compris dans un intervalle allant de 5 à 20 mm.

Dans un mode de réalisation, le greffon présentant une forme tubulaire comprenant une lumière dont la section est circulaire ou sensiblement circulaire non collabable est caractérisé en ce qu'il présente un diamètre externe ou un diamètre équivalent externe compris dans un intervalle allant de 5 à 15 mm.

Le greffon selon la présente invention peut avoir une taille supérieure à la taille physiologique de l'urètre de manière à être coupé à la dimension et la forme souhaitée par le praticien, tout en concevant la courbure de la forme circulaire ou sensiblement circulaire de la lumière de la veine ombilicale.

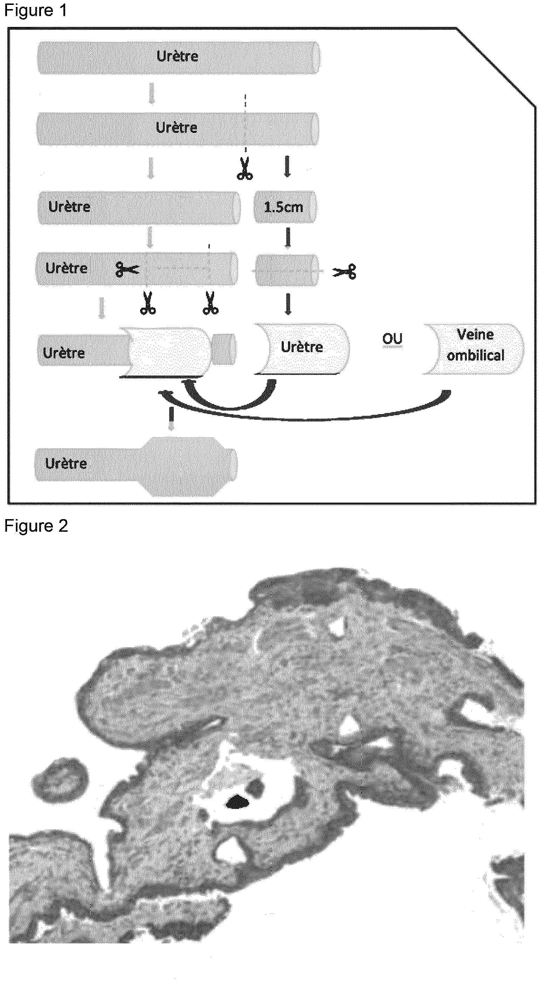

Un exemple de forme partiellement tubulaire est illustré en figure 1.

En particulier, il pourra être découpé en une forme partiellement tubulaire non collabable présentant une courbure adaptée à la reconstruction urétrale.

Dans un mode de réalisation, le greffon selon la présente invention présente une forme partiellement tubulaire non collabable, ledit greffon comprenant au moins une veine ombilicale ou un segment de veine ombilicale disposée de telle sorte que l'épithélium vasculaire est orienté vers la face intérieure de la courbure.

Au sens de la présente invention, on entend par « forme partiellement tubulaire » un tube incomplet ou un quadrilatère conservant la courbure de la forme tubulaire.

Les formes partiellement tubulaires peuvent notamment être substituées aux greffes de muqueuse orale dans le cadre du traitement des sténoses urétrales.

Dans un mode de réalisation, le greffon présentant une forme partiellement tubulaire non collabable est caractérisé en ce que, sur un plan transversal, la distance entre les deux points les plus éloignés de face intérieure de la courbure du greffon est au moins de 5 mm.

Dans un mode de réalisation, le greffon présentant une forme partiellement tubulaire non collabable est caractérisé en ce que, sur un plan transversal, la distance entre les deux points les plus éloignés de face intérieure de la courbure du greffon est au moins de 7 mm.

Dans un mode de réalisation, le greffon présentant une forme partiellement tubulaire non collabable est caractérisé en ce que, sur un plan transversal, la distance entre les deux points les plus éloignés de face intérieure de la courbure du greffon est au moins de 10 mm.

Dans un mode de réalisation, le greffon présentant une forme partiellement tubulaire non collabable est caractérisé en ce que, sur un plan transversal, la distance entre les deux points les plus éloignés de face intérieure de la courbure du greffon est comprise dans un intervalle allant de 2 à 15 mm.

Dans un mode de réalisation, le greffon présentant une forme partiellement tubulaire non collabable est caractérisé en ce que, sur un plan transversal, la distance entre les deux points les plus éloignés de face intérieure de la courbure du greffon est comprise dans un intervalle allant de 8 à 12 mm.

Dans un mode de réalisation, le greffon présentant une forme partiellement tubulaire non collabable est caractérisé en ce que, sur un plan transversal, la distance entre les deux points les plus éloignés de face intérieure de la courbure du greffon est comprise dans un intervalle allant de 2 à 7 mm.

Dans un mode de réalisation, le greffon présentant une forme partiellement tubulaire non collabable est caractérisé en ce que, sur un plan transversal, la distance entre les deux points les plus éloignés de face extérieure du greffon est comprise dans un intervalle allant de 5 à 25 mm.

Dans un mode de réalisation, le greffon présentant une forme partiellement tubulaire non collabable est caractérisé en ce que, sur un plan transversal, la distance entre les deux points les plus éloignés de face extérieure du greffon est comprise dans un intervalle allant de 5 à 20 mm.

Dans un mode de réalisation, le greffon présentant une forme partiellement tubulaire non collabable est caractérisé en ce que, sur un plan transversal, la distance entre les deux points les plus éloignés de face extérieure du greffon est comprise dans un intervalle allant de 5 à 15 mm.

Dans un mode de réalisation, la veine ombilicale est caractérisée en ce qu'elle est d'origine humaine ou animale.

Dans un mode de réalisation, la veine ombilicale est caractérisée en ce qu'elle est d'origine humaine.

Tout matériel biologique humain employé selon la présente invention, en particulier la veine ombilicale, est issu de donneurs consentants en accord avec les exigences des directives Européennes et la Loi Bioéthique. Par exigence sanitaire relative aux dons de tissus et de cellules d'origine humaine, une qualification socio-clinique du donneur et biologique en amont du donneur est systématique. Cette qualification implique une recherche de virus HIV, hépatites B, C, HTLV et de la bactérie tréponème pâle responsable de la syphilis.

Dans un mode de réalisation, un greffon selon la présente invention est caractérisé en ce qu'il comprend au moins un segment de veine ombilicale disposée de telle sorte que l'épithélium vasculaire est orienté vers la lumière.

Au sens de la présente invention, on entend par « segment », une fraction de la veine ombilicale qui a été sectionnée en deux extrémités de telle sorte que la fraction présente une forme tubulaire.

Dans un mode de réalisation, le segment de veine ombilicale correspond à la portion de la veine ombilicale située dans la moitié de la veine ombilicale à proximité du placenta.

Dans un mode de réalisation, le segment de veine ombilicale est caractérisé en ce qu'il correspond à la portion de la veine ombilicale située dans les 15 centimètres les plus proches du placenta.

Dans un mode de réalisation, le segment de veine ombilicale est caractérisé en ce qu'il est issu de la portion de la veine ombilicale située à la jonction avec le placenta.

Cette portion de la veine ombilicale présente l'avantage de permettre d'obtenir un diamètre et une élasticité similaires à ceux de l'urètre.

Dans un mode de réalisation, la veine ombilicale ou le segment de veine ombilicale est caractérisée en ce qu'il est stérile.

Selon la présente invention, on entend par « stérile » une structure exempte de germe, à l'état naturel ou après stérilisation.

La stérilisation pourra être effectuée par toute les méthodes classiquement utilisées par l'Homme du métier.

Dans un mode de réalisation, la stérilisation sera réalisée en irradiation gamma.

Dans un mode de réalisation, la veine ombilicale ou le segment de veine ombilicale est caractérisée en ce qu'il est viro-inactivée.

Par « viro-inactivation », on entend une technique qui permet de réduire fortement ou totalement, et définitivement, la capacité d'action des virus. Ceux-ci, définis comme inactivés, perdent leurs capacités pathogéniques et de réplication par une diminution de leur population de 4 log lors des titrages résiduels qui suivent une ou deux étapes chimiques indépendantes, que ce soit sur des virus enveloppés, ou non enveloppés, ADN ou ARN.

Un tel procédé est par exemple décrit dans le document WO2017/140914 au nom de TBF GENIE TISSULAIRE.

Dans un mode de réalisation, la veine ombilicale ou le segment de veine ombilicale est caractérisé en ce qu'il est viro-inactivé selon les deux étapes de viro-inactivation chimiques du procédé décrit dans WO2017/140914.

Dans un mode de réalisation, la veine ombilicale ou le segment de veine ombilicale est caractérisé en ce qu'il est viro-inactivé selon le protocole tel que décrit ci-après.

Le procédé de viro-inactivation mentionné ci-dessus permet en outre de conserver la tenue de la forme au cours du temps et de conserver le caractère non collabable du greffon.

Dans un mode de réalisation, la veine ombilicale ou le segment de veine ombilicale est caractérisé en ce qu'il est découpé transversalement.

Dans un mode de réalisation, la veine ombilicale ou le segment de veine ombilicale est caractérisé en ce qu'il présente une forme tubulaire.

Dans un mode de réalisation, la veine ombilicale ou le segment de veine ombilicale est caractérisé en ce qu'il présente une forme partiellement tubulaire.

Dans un mode de réalisation, le segment de veine ombilicale est caractérisé en ce qu'il mesure de 1 à 50 cm de longueur.

Dans un mode de réalisation, le segment de veine ombilicale est caractérisé en ce qu'il mesure de 2 à 40 cm de longueur.

Dans un mode de réalisation, le segment de veine ombilicale est caractérisé en ce qu'il mesure de 3 à 30 cm de longueur.

Dans un mode de réalisation, le segment de veine ombilicale est caractérisé en ce qu'il mesure de 4 à 20 cm de longueur.

Dans un mode de réalisation, la veine ombilicale ou le segment de veine ombilicale est caractérisé en ce qu'il est lyophilisé.

Au sens de la présente invention, on entend par « lyophilisation », une technique visant à dessécher un produit préalablement surgelé par sublimation. Plus précisément, le liquide à ôter du produit est dans un premier temps transformé en glace par congélation ; puis par une dessiccation primaire, sous vide, la glace est sublimée ; enfin par une dessiccation secondaire, les molécules d'eau à la surface du produit sont extraites par désorption.

La lyophilisation permet en outre d'augmenter la tenue de la forme au cours du temps et d'augmenter le caractère non collabable du greffon.

Dans un mode de réalisation, le greffon selon l'invention est caractérisé en ce qu'il comprend de la gelée de Wharton.

Dans un mode de réalisation, le greffon selon l'invention est caractérisé en ce qu'il comprend une veine ombilicale ou un segment de veine ombilicale comprenant de la gelée de Wharton.

Dans un mode de réalisation, le greffon selon l'invention est caractérisé en ce qu'il comprend une veine ombilicale ou un segment de veine ombilicale entouré de gelée de Wharton.

Dans un mode de réalisation, le greffon selon l'invention est caractérisé en ce qu'il comprend une épaisseur de gelée de Wharton comprise dans un intervalle allant de 0,3 à 5 mm.

Dans un mode de réalisation, le greffon selon l'invention est caractérisé en ce qu'il comprend une épaisseur de gelée de Wharton comprise dans un intervalle allant de 1 à 4 mm.

Dans un mode de réalisation, le greffon selon l'invention est caractérisé en ce qu'il comprend une épaisseur de gelée de Wharton comprise dans un intervalle allant de 2 à 3,5 mm.

La présence de gelée de Wharton permet en outre d'augmenter la tenue de la forme au cours du temps et d'augmenter le caractère non collabable du greffon.

Dans un mode de réalisation, la veine ombilicale ou le segment de veine ombilicale est caractérisé en ce qu'il comprend de la gelée de Wharton.

Dans un mode de réalisation, la veine ombilicale ou le segment de veine ombilicale est caractérisé en ce qu'il comprend une épaisseur de gelée de Wharton comprise dans un intervalle allant de 0,3 à 5 mm.

Dans un mode de réalisation, la veine ombilicale ou le segment de veine ombilicale est caractérisé en ce qu'il comprend une épaisseur de gelée de Wharton comprise dans un intervalle allant de 1 à 4 mm.

Dans un mode de réalisation, la veine ombilicale ou le segment de veine ombilicale est caractérisé en ce qu'il comprend une épaisseur de gelée de Wharton comprise dans un intervalle allant de 2 à 3,5 mm.

Dans un mode de réalisation, la veine ombilicale ou le segment de veine ombilicale sont caractérisés en ce qu'ils n'ont pas subi de traitement par une solution aqueuse d'aldéhyde.

On entend par aldéhyde notamment les aldéhydes cités dans la demande US3988782, à savoir notamment le glutaraldéhyde, l'amidon dialdéhyde, le formaldéhyde ou le glyoxal.

On entend par solution aqueuse d'aldéhyde une solution comprenant de 0,1 à 3,0 % en masse d'aldéhyde.

Dans un mode de réalisation, la veine ombilicale ou le segment de veine ombilicale sont caractérisés en ce qu'ils n'ont pas subi de traitement par une solution de glutaraldéhyde.

Dans un mode de réalisation, la veine ombilicale ou le segment de veine ombilicale sont caractérisés en ce que leurs protéines ne sont pas réticulées.

Dans un mode de réalisation, la veine ombilicale ou le segment de veine ombilicale sont caractérisés en ce qu'ils ne sont pas siliconés.

On entend par veine ombilicale ou le segment de veine ombilicale siliconé, une veine ombilicale ou un segment de veine ombilicale ayant subi un traitement par un bain de silicone en solution à 4 à 5 % massique dans un solvant organique comme le toluène.

Au sens de la présente invention, on entend par « non collabable » une déformabilité limitée en réponse à l'application d'une force mécanique et que notamment il ne présente pas de collapsus. En d'autres termes, l'aire de la section droite est sensiblement constante quelles que soient les pressions exercées.

A la différence, une veine utilisée en milieu humide par exemple après prélèvement lors d'une autogreffe ne peut être considérée comme non collabable : prise par exemple en l'une de ses extrémités ou en son centre, elle se déforme par l'effet de la gravité et sa forme tubulaire n'est pas conservée.

Le greffon selon l'invention présente néanmoins une souplesse suffisante afin de s'adapter aux gestes techniques chirurgicaux du praticien.

Cette souplesse du greffon selon l'invention est suffisante pour notamment accompagner les mouvements d'un patient ayant par exemple reçu pour greffe un greffon selon l'invention dans le cadre du traitement d'une sténose urétrale, en particulier chez l'homme.

Il est cependant suffisamment rigide pour contenir le débit mictionnel.

Dans un mode de réalisation, le greffon selon l'invention est caractérisé en ce qu'il présente une rigidité suffisante pour contenir le débit mictionnel.

Au sens de la présente invention, on entend par « reconstruction urétrale » toute technique chirurgicale visant à remplacer et/ou prolonger tout ou partie de l'urètre.

Dans un mode de réalisation, le greffon est caractérisé en ce qu'il est utilisable pour la reconstruction urétrale chez l'Homme ou chez l'animal.

Dans un mode de réalisation, le greffon est caractérisé en ce qu'il est utilisable pour la reconstruction urétrale chez l'Homme.

Dans un mode de réalisation, le greffon est caractérisé en ce qu'il est utilisable pour la reconstruction urétrale dans le traitement des sténoses urétrales, des hypospadias et/ou dans le cadre d'une metaïdoplastie.

Dans un mode de réalisation, le greffon est utilisable pour la reconstruction urétrale dans le traitement des sténoses urétrales.

Dans un mode de réalisation, le greffon selon l'invention est caractérisé en ce qu'il est apte à être utilisé dans le traitement des sténoses urétrales, des hypospadias et/ou dans le cadre d'une metaïdoplastie chez l'Homme ou l'animal.

Dans un mode de réalisation, le greffon selon l'invention est caractérisé en ce qu'il est apte à être utilisé dans le traitement des sténoses urétrales, des hypospadias et/ou dans le cadre d'une metaïdoplastie chez l'Homme.

Dans un mode de réalisation, le greffon selon l'invention est caractérisé en ce qu'il est apte à être utilisé dans le traitement des sténoses urétrales chez l'Homme ou l'animal.

Dans un mode de réalisation, le greffon selon l'invention est caractérisé en ce qu'il est apte à être utilisé dans le traitement des sténoses urétrales chez l'Homme.

L'aptitude à être utilisé dans le traitement des sténoses urétrales, des hypospadias ou dans le cadre d'une metaïdoplastie sera notamment conditionnée par capacité du tissu à être implanté en lieu et place de la portion de l'urètre à remplacer ou à prolonger en particulier par sa forme, son étanchéité et/ou sa souplesse compatible avec celle de l'urètre physiologique, et/ou sa capacité d'intégration en particulier pour la vascularisation et/ou la cicatrisation de l'urètre, et/ou la capacité à permettre la colonisation de l'épithélium urétral.

Dans un mode de réalisation, la sténose urétrale est une sténose iatrogène, infectieuse, traumatique ou congénitale.

Dans un mode de réalisation, le greffon selon l'invention est caractérisé en ce qu'il est apte à maintenir et/ou restaurer un débit mictionnel supérieur à 16 mL/s.

Dans un mode de réalisation, le greffon selon la présente invention est un urètre artificiel.

Au sens de la présente invention, on entend par « urètre artificiel » un tissu transplantable destiné à remplacer tout ou partie de l'urètre.

L'invention concerne également un procédé de préparation d'un greffon utilisable pour la reconstruction urétrale selon l'invention comprenant les étapes de :
a) on dispose d'au moins une veine ombilicale ou un segment de celle-ci ;
b) on lyophilise ladite veine ombilicale ou le segment de celle-ci,
c) on obtient un greffon utilisable pour la reconstruction urétrale,
ledit procédé ne comprenant pas d'étape d'aplatissement de la veine ombilicale.

Dans un mode de réalisation, le procédé selon la présente invention est caractérisé en ce que le greffon obtenu à l'étape c) présente une forme tubulaire comprenant une lumière dont la section est circulaire ou sensiblement circulaire non collabable, ledit greffon comprenant au moins une veine ombilicale ou un segment de veine ombilicale disposée de telle sorte que l'épithélium vasculaire est orienté vers la lumière.

Dans un mode de réalisation, le procédé selon la présente invention est caractérisé en ce que le greffon obtenu à l'étape c) présente une forme tubulaire telle que précédemment définie.

Dans un mode de réalisation, le procédé selon la présente invention est caractérisé en ce que le greffon obtenu à l'étape c) présente une forme partiellement tubulaire non collabable, ledit greffon comprenant au moins une veine ombilicale ou un segment de veine ombilicale disposée de telle sorte que l'épithélium vasculaire est orienté vers la face intérieure de la courbure.

Dans un mode de réalisation, le procédé selon la présente invention est caractérisé en ce que le greffon obtenu à l'étape c) présente une forme partiellement telle que précédemment définie.

Dans un mode de réalisation, le procédé de préparation d'un greffon selon l'invention est caractérisé en ce qu'il ne comprend pas d'étape de traitement par une solution aqueuse d'aldéhyde et/ou une solution organique de silicone.

On entend par solution aqueuse d'aldéhyde une solution comprenant de 0,1 à 3,0 % en masse d'aldéhyde.

On entend par aldéhyde notamment les aldéhydes cités dans la demande US3988782, à savoir notamment le glutaraldéhyde, l'amidon dialdéhyde, le formaldéhyde ou le glyoxal.

On entend par solution organique de silicone une solution de silicone à 4 à 5 % massique dans un solvant organique comme le toluène.

Dans un mode de réalisation, le procédé de préparation d'un greffon selon l'invention est caractérisé en ce qu'il ne comprend pas d'étape de réticulation.

Dans un mode de réalisation, le procédé de préparation d'un greffon selon l'invention est caractérisé en ce qu'il ne comprend pas d'étape de traitement par une solution d'aldéhyde.

Dans un mode de réalisation, le procédé de préparation d'un greffon selon l'invention est caractérisé en ce qu'il ne comprend pas d'étape de traitement par une solution organique de silicone.

Dans un mode de réalisation, le greffon obtenu à l'étape c) est caractérisé en ce qu'il est implantable sans réhydratation préalable.

La veine ombilicale dont on dispose à l'étape a) est issue d'un cordon ombilical. Ce cordon ombilical est obtenu après l'accouchement d'un donneur proprement informé et consentant en accord avec les exigences des directives Européennes et la Loi Bioéthique. Par exigence sanitaire relative aux dons de tissus et de cellules d'origine humaine, une qualification socio-clinique du donneur et biologique en amont du donneur est systématique. Cette qualification implique une recherche de virus HIV, hépatites B, C, HTLV et de la bactérie tréponème pâle responsable de la syphilis. Le cordon ombilical est avantageusement placé dans une boîte stérile, comprenant une solution de NaCl à +4°C, et transporté à même température. Si les données cliniques du donneur sont conformes aux exigences relatives au don tissulaire, et que l'analyse sanguine de recherche de bactéries et virus se révèle négative, le tissu éligible peut subir le procédé de préparation.

Dans un mode de réalisation, les segments de veine dont on dispose à l'étape a) ont une longueur de 1 à 50 cm.

Dans un mode de réalisation, les segments de veine dont on dispose à l'étape a) ont une longueur de 2 à 40 cm.

Dans un mode de réalisation, les segments de veine dont on dispose à l'étape a) ont une longueur de 3 à 30 cm.

Dans un mode de réalisation, les segments de veine dont on dispose à l'étape a) ont une longueur de 4 à 15 cm.

Dans un mode de réalisation, le procédé de préparation d'un greffon selon l'invention comprend une étape a₁) après l'étape a), de découpe de la gelée de Wharton.

Un mandrin, ou guide ou support rigide, de diamètre adapté est introduit dans la lumière de la veine. Il est avantageusement utilisé comme mandrin, ou guide ou support rigide, une aiguille à bout rond, avec un chas à une extrémité. Un diamètre adapté pour le mandrin, ou guide ou support rigide est d'environ 3 millimètres.

Dans un mode de réalisation, la découpe de la gelée de Wharton est réalisée de telle sorte qu'il persiste une épaisseur résiduelle de gelée de Wharton d'environ 1 à 5 millimètres.

Dans un mode de réalisation, la découpe de la gelée de Wharton est réalisée de telle sorte qu'il persiste une épaisseur résiduelle de gelée de Wharton d'environ 2 à 4 millimètres.

Dans un mode de réalisation, la découpe de la gelée de Wharton est réalisée de telle sorte qu'il persiste une épaisseur résiduelle de gelée de Wharton d'environ 3 millimètres.

Dans un mode de réalisation, la découpe de la gelée de Wharton est réalisée au scalpel, de telle sorte qu'il persiste une épaisseur résiduelle de gelée de Wharton d'environ 1 à 5 millimètres.

Dans un mode de réalisation, la découpe de la gelée de Wharton est réalisée au scalpel, de telle sorte qu'il persiste une épaisseur résiduelle de gelée de Wharton d'environ 2 à 4 millimètres.

Dans un mode de réalisation, la découpe de la gelée de Wharton est réalisée au scalpel, de telle sorte qu'il persiste une épaisseur résiduelle de gelée de Wharton d'environ 3 millimètres.

Dans un mode de réalisation, le procédé de préparation d'un greffon selon l'invention est réalisé à température ambiante, environ 20°C.

Dans un mode de réalisation, le procédé de préparation d'un greffon selon l'invention comprend une étape a₂) après l'étape a), ou après l'étape a₁) lorsqu'elle est mise en oeuvre, et avant l'étape b, au moins une étape de traitement de ladite veine ombilicale avec une solution de tréhalose.

Dans un mode de réalisation, la solution de tréhalose de l'étape a₂) présente une teneur en tréhalose comprise dans un intervalle allant de 1 à 20% (v/v) en volume de tréhalose par rapport au volume total de la solution.

Dans un mode de réalisation, la solution de tréhalose de l'étape a₂) présente une teneur en tréhalose comprise dans un intervalle allant de 5 à 15% (v/v) en volume de tréhalose par rapport au volume total de la solution.

Dans un mode de réalisation, la solution de tréhalose de l'étape a₂) présente une teneur en tréhalose comprise dans un intervalle allant de 8 à 12% (v/v) en volume de tréhalose par rapport au volume total de la solution.

Dans un mode de réalisation, le procédé de préparation d'un greffon selon l'invention est caractérisé en ce qu'il comprend en outre, avant l'étape b) une étape a₃) de viro-inactivation.

On entend par « viro-inactivation », une technique qui permet de réduire fortement ou totalement, et définitivement, la capacité d'action des virus. Ceux-ci, définis comme inactivés, perdent leurs capacités pathogéniques et de réplication par une diminution de leur population de 4 log lors des titrages résiduels qui suivent une ou deux étapes chimiques indépendantes, que ce soit sur des virus enveloppés, ou non enveloppés, ADN ou ARN.

Un procédé de viro-inactivation d'un guide implantable pour la régénération tissulaire comprenant un vaisseau ombilical est décrit dans le document WO2018083353.

Dans un mode de réalisation, le procédé de préparation d'un greffon selon l'invention est caractérisé en ce qu'il comprend en outre une étape a₃) de viro-inactivation selon le procédé de viro-inactivation décrit dans WO2018083353.

Dans un mode de réalisation, le procédé de préparation d'un greffon selon l'invention est caractérisé en ce que le traitement de viro-inactivation comprend au moins deux étapes successives de traitement par un agent de viro-inactivation chimique.

Par commodité, de manière non limitative, il sera fait référence aux étapes x et y pour désigner ces deux étapes successives de traitement par un agent de viro-inactivation chimique.

Dans un mode de réalisation, l'agent de viro-inactivation chimique est choisi dans le groupe constitué par les alcools et/ou les peroxydes.

Dans un mode de réalisation, la première étape de traitement x est effectuée par un agent de viro-inactivation choisi dans le groupe constitué par les alcools.

Selon un mode de réalisation, le premier agent de viro-inactivation est l'éthanol à une teneur en alcool comprise entre 50 % et 80 % (v/v).

Dans un mode de réalisation, l'agent de viro-inactivation choisi dans le groupe constitué par les alcools est l'éthanol à 70 % (v/v).

Dans un mode de réalisation, la première étape de traitement x est effectuée pendant une durée de 60 minutes.

Dans un mode de réalisation, le deuxième agent de viro-inactivation est le peroxyde d'hydrogène.

Dans un mode de réalisation, le peroxyde d'hydrogène est sous une forme choisie parmi une solution aqueuse et un gaz.

Dans un mode de réalisation, le peroxyde d'hydrogène est sous forme aqueuse dans une concentration comprise entre 3 % et 30 % (p/v).

Dans un mode de réalisation, la deuxième étape de viro-inactivation chimique y comporte deux sous-étapes de traitement, q et r, par le peroxyde d'hydrogène :
- une première sous-étape de traitement q s'effectuant avec une solution de peroxyde d'hydrogène à 30 % (p/v pendant 15 minutes ;
- une deuxième sous-étape de traitement r s'effectuant avec une solution de peroxyde d'hydrogène à 3 % (p/v) pendant 60 minutes.

Dans un mode de réalisation, le procédé de préparation d'un greffon selon l'invention est caractérisé en ce qu'il comporte en outre au moins une étape lors de laquelle le segment est lavé à l'eau purifiée entre les étapes x et y de viro-inactivation chimique.

Le procédé de préparation d'un greffon selon l'invention est caractérisé en ce qu'il comporte en outre au moins une étape d'ajustement de pH de la solution dans laquelle se trouve le segment viro-inactivé avant l'étape b).

Le procédé de préparation d'un greffon selon l'invention est, caractérisé en ce qu'il comporte en outre au moins une étape d'ajustement de pH à 8,5 de la solution dans laquelle se trouve le segment viro-inactivé avant l'étape b).

Le procédé de préparation d'un greffon selon l'invention est, caractérisé en ce qu'il comporte en outre au moins une étape d'ajustage de pH de la solution dans laquelle se trouve le segment viro-inactivé, suivie d'au moins une étape de mise en tampon du segment viro-inactivé avant l'étape b).

Selon un mode de réalisation, cette au moins une étape de mise en tampon est définie par deux bains successifs de solution tampon de PBS (tampon phosphate salin).

Selon un mode de réalisation, cette étape de mise en solution tampon est suivie de deux étapes de lavage en bains d'eau purifiée.

Le procédé de préparation d'un greffon selon l'invention est, caractérisé en ce que l'étape b) de lyophilisation est effectuée selon les étapes m et n suivantes :
m) une congélation en deux étapes m' et m" :
   - la première étape de congélation, m', s'effectuant à une température d'acclimatation choisie pour ne pas endommager l'intégralité structurelle, fonctionnelle et biologique du segment viro-inactivé ;
   - la deuxième étape de congélation, m", s'effectuant à la température finale de congélation qui est inférieure à la température d'acclimatation ;
n) une lyophilisation en deux étapes principales, dites primaire, n', et secondaire, n" :
   - l'étape de lyophilisation primaire n' s'effectuant par application d'un vide à environ 200 micro bars et d'un profil de température ascendant ;
   - l'étape de lyophilisation secondaire n" s'effectuant par application d'un vide à environ 50 micro-bars et d'un profil de température descendant.

Le profil de température ascendant est avantageusement un profil selon lequel la température de lyophilisation est initialement réglée à une température initiale basse puis augmentée vers une température finale de lyophilisation primaire, en une ou plusieurs étapes de températures ascendantes intermédiaires. Le profil de température descendant est avantageusement un profil selon lequel la température de lyophilisation est initialement réglée à une température supérieure à la température finale de l'étape de lyophilisation primaire, puis qui est ensuite descendue vers une température finale de lyophilisation secondaire supérieure à la température initiale de l'étape de lyophilisation primaire.

Ces conditions de lyophilisation permettent d'obtenir un produit, qui est un segment viro-inactivé et lyophilisé, qui est notamment caractérisé en ce qu'il présente une teneur en eau résiduelle à température ambiante inférieure à 5%.

Dans un mode de réalisation, le procédé de préparation du greffon selon l'invention est caractérisé en ce qu'il comprend en outre ultérieurement à l'étape b) ou à l'étape c) une étape de stérilisation.

Dans un mode de réalisation, le procédé de préparation du greffon selon l'invention est caractérisé en ce qu'il comprend en outre ultérieurement à l'étape b) ou à l'étape c) une étape de stérilisation aux rayonnements gamma.

Dans un mode de réalisation, le procédé de préparation du greffon selon l'invention est caractérisé en ce qu'il comprend en outre ultérieurement à l'étape b) une étape b₁) de découpe longitudinale et/ou transversale du greffon.

Dans un mode de réalisation, le procédé de préparation du greffon selon l'invention est caractérisé en ce qu'il comprend en outre ultérieurement à l'étape b) une étape b₁) de découpe longitudinale et transversale du greffon de manière à obtenir un greffon présentant une forme partiellement tubulaire non collabable.

Dans un mode de réalisation, le procédé de préparation du greffon selon l'invention est caractérisé en ce qu'il comprend en outre ultérieurement à l'étape b) une étape b₁) de découpe longitudinale et transversale du greffon de manière à obtenir un quadrilatère conservant la courbure adaptée à celle de l'urètre.

Dans un mode de réalisation, le procédé de préparation du greffon selon l'invention est caractérisé en ce qu'il comprend en outre ultérieurement à l'étape b) une étape b₁) de découpe longitudinale et transversale du greffon de manière à obtenir un quadrilatère conservant la courbure adaptée à celle de l'urètre et l'épithélium vasculaire orienté dans la face intérieure de la courbure.

Dans un mode de réalisation, l'étape b₁) de découpe est caractérisée en ce qu'elle est effectuée au moyen d'un instrument et/ou d'un appareil choisi dans le groupe comprenant : un scalpel, une paire de ciseaux chirurgicaux, un microtome, un bistouri électrique, une pince chirurgicale, un laser chirurgical, une lame de rasoir, une scie oscillante, une perforatrice à biopsie et/ou une pince coupante.

L'invention concerne également un procédé de préparation d'un greffon utilisable pour la reconstruction urétrale, ledit greffon présentant une forme partiellement tubulaire non collabable, et comprenant au moins une veine ombilicale ou un segment de veine ombilicale disposée de telle sorte que l'épithélium vasculaire est orienté vers la face intérieure de la courbure, comprenant les étapes de :
a) on dispose d'au moins une veine ombilicale ou un segment de celle-ci,
b) on lyophilise ladite veine ombilicale ou le segment de celle-ci,
b₁) on procède à la découpe longitudinale et/ou transversale du greffon,
c) on obtient un greffon utilisable pour la reconstruction urétrale présentant une forme partiellement tubulaire non collabable, et comprenant au moins une veine ombilicale ou un segment de veine ombilicale disposée de telle sorte que l'épithélium vasculaire est orienté vers la face intérieure de la courbure,
ledit procédé ne comprenant pas d'étape d'aplatissement de la veine ombilicale.

Selon un mode de réalisation, le greffon selon l'invention est conditionné en conditions stériles.

Selon un mode de réalisation, le greffon selon l'invention est conditionné selon l'invention en conditions stériles, en double sachets.

Selon un mode de réalisation, le greffon selon l'invention qui est emballé, subit une dernière étape de stérilisation qui est une irradiation gamma.

Le procédé de préparation d'un greffon selon l'invention est, caractérisé en ce qu'il comporte en outre une dernière étape de stérilisation du guide implantable non collabable viro-inactivé et lyophilisé, par exposition de ce dernier à des rayonnements gamma à 25-32 kGy.

La présente invention concerne également le greffon utilisable pour la reconstruction urétrale présentant une forme tubulaire comprenant une lumière dont la section est circulaire ou sensiblement circulaire non collabable, ou présentant une forme partiellement tubulaire non collabable, ledit greffon comprenant au moins une veine ombilicale ou un segment de veine ombilicale disposée de telle sorte que l'épithélium vasculaire est orienté vers la lumière lorsqu'il présente une forme tubulaire ou vers la face intérieure de la courbure lorsqu'il présente une forme partiellement tubulaire selon la présente invention, pour son utilisation chirurgicale.

La présente invention concerne également greffon utilisable pour la reconstruction urétrale présentant une forme tubulaire comprenant une lumière dont la section est circulaire ou sensiblement circulaire non collabable, ou présentant une forme partiellement tubulaire non collabable, ledit greffon comprenant au moins une veine ombilicale ou un segment de veine ombilicale disposée de telle sorte que l'épithélium vasculaire est orienté vers la lumière lorsqu'il présente une forme tubulaire ou vers la face intérieure de la courbure lorsqu'il présente une forme partiellement tubulaire selon la présente invention pour son utilisation chirurgicale dans le traitement des sténoses urétrales et/ou des hypospadias et/ou dans le cadre d'une metaïdoplastie.

Dans un mode de réalisation, la sténose urétrale est une sténose iatrogène, traumatique ou congénitale.

La figure 1 est une représentation schématique du protocole opératoire de greffe pour la reconstruction urétrale à l'aide du greffon selon l'invention tel que présenté à l'exemple 2.

La figure 2 est une photographie de la coupe histologique de la biopsie réalisée par endoscopie trois semaines après la greffe, dans les conditions selon l'exemple 2 pour la condition témoin (autogreffe).

La figure 3 est une photographie de la coupe histologique de la biopsie réalisée par endoscopie trois semaines après la greffe, dans les conditions selon l'exemple 2 pour la condition selon l'invention (allogreffe de greffon selon l'invention).

La figure 4 est une photographie de la coupe histologique de la biopsie réalisée après mise à mort du lapin greffé, six semaines après la greffe, dans les conditions selon l'exemple 2 pour la condition témoin (autogreffe).

La figure 5 est une photographie de la coupe histologique de la biopsie réalisée après mise à mort du lapin greffé, six semaines après la greffe, dans les conditions selon l'exemple 2 pour la condition selon l'invention (allogreffe de greffon selon l'invention).

Dans les exemples, tous les pourcentages sont donnés en poids sauf indication contraire, la température est exprimée en degré Celsius sauf indication contraire, les essais sont réalisés à température ambiante sauf indication contraire, et la pression est la pression atmosphérique sauf indication contraire.

### EXEMPLES

### Exemple 1: Préparation d'un greffon utilisable pour la reconstruction urétrale selon la présente invention.

Un donneur proprement informé et consentant en accord avec les exigences de la Déclaration d'Helsinki offre en don le cordon ombilical issu d'un accouchement. Par exigence sanitaire relative aux dons de tissus et de cellules d'origine humaine, une qualification en amont du donneur est systématique. Cette qualification implique une recherche de virus HIV, hépatites B, C, HTLV et de la bactérie tréponème pâle responsable de la syphilis.

Le cordon ombilical est récupéré le plus tôt possible en salle d'accouchement. Il est avantageusement placé dans une boite stérile, comprenant une solution de NaCl à +4°C.

Au laboratoire, en salle stérile, la procédure suivante est appliquée :

Les vaisseaux sanguins du cordon ombilical sont identifiés et séparés les uns des autres par dissection.

La veine est découpée en un segment d'une longueur de 15 centimètres.

Le segment est rincé et hydraté dans des bains d'eau purifiée successifs, sous agitation douce, pendant 4 heures.

Le segment est congelé à sec à une température de -80°C.

Lors du traitement chimique, le segment est décongelé à l'air libre, à température ambiante, pendant une durée de 5 minutes. Cette étape de congélation, suivie d'une décongélation, assure une décellularisation importante du tissu biologique.

Le segment subit une succession de bains assurant un traitement chimique de celui-ci. Ce traitement a pour objectif une désinfection de la veine, et tout particulièrement sa viro-inactivation. Une agitation linéaire douce du milieu liquide est appliquée lors de chaque bain afin d'assurer une pénétration homogène des solvants dans les tissus.

Dans un premier temps, le segment est déposé dans un bain d'eau purifiée à température ambiante pendant environ 3 heures. Cette étape assure tant la fin de la décongélation du tissu physiologique, qu'une étape de lyse cellulaire par pression osmotique.

Puis, le segment est transféré dans un bain décontaminant composé d'éthanol 70 % v/v à température ambiante pendant environ 1 heure.

Un lavage est effectué dans de l'eau purifiée pendant environ 15 minutes à température ambiante pour retirer l'éthanol.

Afin d'assurer la seconde étape de traitement décontaminant, le segment est transféré dans un bain composé de peroxyde d'hydrogène à 30 % w/v à température ambiante pendant environ 15 minutes.

Puis, le segment est transféré dans un bain décontaminant composé de peroxyde d'hydrogène à 3 % w/v à température ambiante pendant environ 1 heure. Le segment obtenu est viro-inactivé.

L'action chimique appliquée au segment viro-inactivé est ensuite neutralisée, dans au moins un bain comportant de l'hydroxyde de sodium dilué autour d'un pH de 8,5. Le traitement d'au moins un bain de neutralisation s'effectue à température ambiante pendant environ 15 minutes.

Afin d'assurer un rééquilibrage du pH et pour éliminer au mieux les résidus organiques se détachant du tissu d'intérêt, le segment viro-inactivé est transféré dans au moins un bain de tampon physiologique (PBS), afin d'assurer son rééquilibrage physiologique. Le au moins un bain s'effectue à température ambiante pendant environ 15 minutes.

Enfin, le segment viro-inactivé est transféré dans deux bains successifs à l'eau purifiée, à température ambiante, pendant au moins 15 minutes et jusqu'à environ 1 heure.

A cette étape du procédé, on peut considérer que le segment obtenu selon ce traitement chimique est un segment en grande partie désinfecté, notamment viro-inactivé, et décellularisé.

Le segment est alors immergé dans un tube contenant une solution de tréhalose à une concentration de 10% en volume dans l'eau.

Le segment viro-inactivé et immergé dans une solution de tréhalose est transféré dans un lyophilisateur, où une étape de congélation suivie d'une étape de lyophilisation sont effectuées selon les modalités suivantes :
- Congélation :
   ∘ La première étape de congélation s'effectue à une température d'acclimatation choisie pour ne pas endommager l'intégrité structurelle, fonctionnelle et biologique du segment viro-inactivé ;
   ∘ la deuxième étape de congélation s'effectue à la température finale de congélation qui est inférieure à la température d'acclimatation ;
- Lyophilisation :
   ∘ Une étape de lyophilisation primaire s'effectue par application d'un vide à environ 200 micro-bars et par application d'un profil de température ascendant ;
   ∘ Une étape secondaire de lyophilisation s'effectue par application d'un vide à environ 50 micro-bars et par application d'un profil de température descendant.

Suite à cette étape de lyophilisation, le segment est un segment désinfecté, notamment viro-inactivé, décellularisé, lyophilisé.

Le segment est ensuite conditionné dans son flacon bouché en conditions stériles en double sachets conservable 5 ans à température ambiante.

Une dernière étape de stérilisation du segment est effectuée par exposition de celui-ci à des rayonnements gamma à 25-32 kGrays.

Le produit ainsi obtenu est prêt à l'utilisation réhydraté en tant que greffon pour la reconstruction urétrale.

### Exemple 1 .2 : Préparation d'un greffon de forme partiellement tubulaire utilisable pour la reconstruction urétrale selon la présente invention.

Un donneur proprement informé et consentant en accord avec les exigences de la Déclaration d'Helsinki offre en don le cordon ombilical issu d'un accouchement. Par exigence sanitaire relative aux dons de tissus et de cellules d'origine humaine, une qualification en amont du donneur est systématique. Cette qualification implique une recherche de virus HIV, hépatites B, C, HTLV et de la bactérie tréponème pâle responsable de la syphilis.

Le cordon ombilical est récupéré le plus tôt possible en salle d'accouchement. Il est avantageusement placé dans une boite stérile, comprenant une solution de NaCl à +4°C.

Au laboratoire, en salle stérile, la procédure suivante est appliquée :
Les vaisseaux sanguins du cordon ombilical sont identifiés et séparés les uns des autres par dissection.

La veine est découpée en un segment d'une longueur de 15 centimètres.

Le segment est rincé et hydraté dans des bains d'eau purifiée successifs, sous agitation douce, pendant 4 heures.

Le segment est congelé à sec à une température de -80°C.

Lors du traitement chimique, le segment est décongelé à l'air libre, à température ambiante, pendant une durée de 5 minutes. Cette étape de congélation, suivie d'une décongélation, assure une décellularisation importante du tissu biologique.

Le segment subit une succession de bains assurant un traitement chimique de celui-ci. Ce traitement a pour objectif une désinfection de la veine, et tout particulièrement sa viro-inactivation. Une agitation linéaire douce du milieu liquide est appliquée lors de chaque bain afin d'assurer une pénétration homogène des solvants dans les tissus.

Dans un premier temps, le segment est déposé dans un bain d'eau purifiée à température ambiante pendant environ 3 heures. Cette étape assure tant la fin de la décongélation du tissu physiologique, qu'une étape de lyse cellulaire par pression osmotique.

Puis, le segment est transféré dans un bain décontaminant composé d'éthanol 70 % v/v à température ambiante pendant environ 1 heure.

Un lavage est effectué dans de l'eau purifiée pendant environ 15 minutes à température ambiante pour retirer l'éthanol.

Afin d'assurer la seconde étape de traitement décontaminant, le segment est transféré dans un bain composé de peroxyde d'hydrogène à 30 % w/v à température ambiante pendant environ 15 minutes.

Puis, le segment est transféré dans un bain décontaminant composé de peroxyde d'hydrogène à 3 % w/v à température ambiante pendant environ 1 heure. Le segment obtenu est viro-inactivé.

L'action chimique appliquée au segment viro-inactivé est ensuite neutralisée, dans au moins un bain comportant de l'hydroxyde de sodium dilué autour d'un pH de 8,5. Le traitement d'au moins un bain de neutralisation s'effectue à température ambiante pendant environ 15 minutes.

Afin d'assurer un rééquilibrage du pH et pour éliminer au mieux les résidus organiques se détachant du tissu d'intérêt, le segment viro-inactivé est transféré dans au moins un bain de tampon physiologique (PBS), afin d'assurer son rééquilibrage physiologique. Le au moins un bain s'effectue à température ambiante pendant environ 15 minutes.

Enfin, le segment viro-inactivé est transféré dans deux bains successifs à l'eau purifiée, à température ambiante, pendant au moins 15 minutes et jusqu'à environ 1 heure.

A cette étape du procédé, on peut considérer que le segment obtenu selon ce traitement chimique est un segment en grande partie désinfecté, notamment viro-inactivé, et décellularisé.

Le segment viro-inactivé et immergé dans une solution de tréhalose est transféré dans un lyophilisateur, où une étape de congélation suivie d'une étape de lyophilisation sont effectuées selon les modalités suivantes :
- Congélation :
   ∘ La première étape de congélation s'effectue à une température d'acclimatation choisie pour ne pas endommager l'intégrité structurelle, fonctionnelle et biologique du segment viro-inactivé ;
   ∘ la deuxième étape de congélation s'effectue à la température finale de congélation qui est inférieure à la température d'acclimatation ;
- Lyophilisation :
   ∘ Une étape de lyophilisation primaire s'effectue par application d'un vide à environ 200 micro-bars et par application d'un profil de température ascendant ;
   ∘ Une étape secondaire de lyophilisation s'effectue par application d'un vide à environ 50 micro-bars et par application d'un profil de température descendant.

Suite à cette étape de lyophilisation, le segment est un segment désinfecté, notamment viro-inactivé, décellularisé et lyophilisé.

Le segment désinfecté, notamment viro-inactivé, décellularisé et lyophilisé est découpé au scalpel, longitudinalement et transversalement de manière à obtenir un quadrilatère d'environ 2 cm de côté, conservant la courbure adaptée à celle de l'urètre et l'épithélium vasculaire orienté dans la face intérieure de la courbure.

Une dernière étape de stérilisation du quadrilatère obtenu est effectuée par exposition de celui-ci à des rayonnements gamma à 25-32 kGrays.

On obtient un greffon de forme partiellement tubulaire utilisable pour la reconstruction urétrale selon la présente invention.

### Exemple 2 : Reconstruction urétrale à l'aide du greffon selon la présente invention,

### Protocole opératoire de greffe

Six lapins mâles ont été opérés sous anesthésie générale, l'intervention consistant en une incision de la peau du fourreau de la verge puis en une dissection complète de l'urètre, jusqu'à l'urètre périnéal. L'urètre disséqué est obtenu. Les lapins ont été divisés en deux groupes.

A l'extrémité de l'urètre disséqué du premier groupe de lapin, un segment d'urètre tubulaire (1) de 1,5 cm est découpé, le reste de l'urètre sectionné (1) est réservé.

Le segment d'urètre tubulaire (1) est découpé longitudinalement sur toute sa longueur. On obtient un patch de segment d'urètre en forme de quadrilatère. Ce patch servira de témoin d'une allogreffe d'urètre.

Un autre urètre est disséqué, amputé d'un segment d'urètre tubulaire de 1,5 cm, puis l'urètre sectionné (2) est réservé, le segment d'urètre tubulaire (2) n'est pas conservé.

En parallèle pour le deuxième groupe de lapin, un segment tubulaire de 1,5 cm est découpé dans le greffon obtenu selon l'exemple 1, puis découpé longitudinalement sur toute sa longueur. On obtient un greffon selon l'exemple 1 présentant une forme partiellement tubulaire.

Les urètres sectionnés (1) et (2) sont incisés longitudinalement à une de leurs extrémités sur une longueur 1,5 cm. On obtient deux urètres incisés, respectivement (1) et (2).

Le patch de segment d'urètre est suturé bord à bord sur l'urètre incisé (1) (témoin : allogreffe) et le greffon selon l'exemple 1 présentant une forme partiellement tubulaire est suturé bord à bord sur l'urètre incisé (2) selon les techniques classiques de reconstruction de l'urètre en une seule étape, telles que celles employées lors de greffe de muqueuse orale.

Les urètres reconstruits obtenus sont regreffés sur chacun des lapins. L'étanchéité est constatée pour les deux greffons.

Le protocole opératoire est schématisé en figure 1.

### Analyse

Trois semaines après la greffe, une analyse intermédiaire est réalisée par endoscopie urétrale sous anesthésie générale. Une analyse semi quantitative selon les normes ISO 10993-6:2016 et la norme ISO 10993-10:2021 et une analyse histologique (hématoxyline-éosine) après biopsie urétrale sont réalisées.

Les résultats de l'analyse semi quantitative sont présentés dans le Tableau 1.

**Tableau 1**

| | | Témoin : allogreffe | Greffon selon l'exemple 1 |
|---|---|---|---|
| Types de cellules inflammatoires | Lymphocytes | 0 | 0,5 |
| | Polymorphonucléaires | 0,5 | 0,5 |
| | Cellules plasmatiques | 0,5 | 0 |
| | Macrophages | 0 | 0 |
| | Cellule géante multinucléée | 0 | 0 |
| Réponse lymphocytaire | Fibrose | 0 | 0 |
| | Neo-vascuralisation | 0 | 0 |
| | Congestion vasculaire | 0 | 0,5 |
| | Oedème | 0 | 0,5 |
| | Necrose | 0 | 0 |
| | Infiltration graisseuse | 0 | 0 |
| | Epithélium | 0 | 0 |

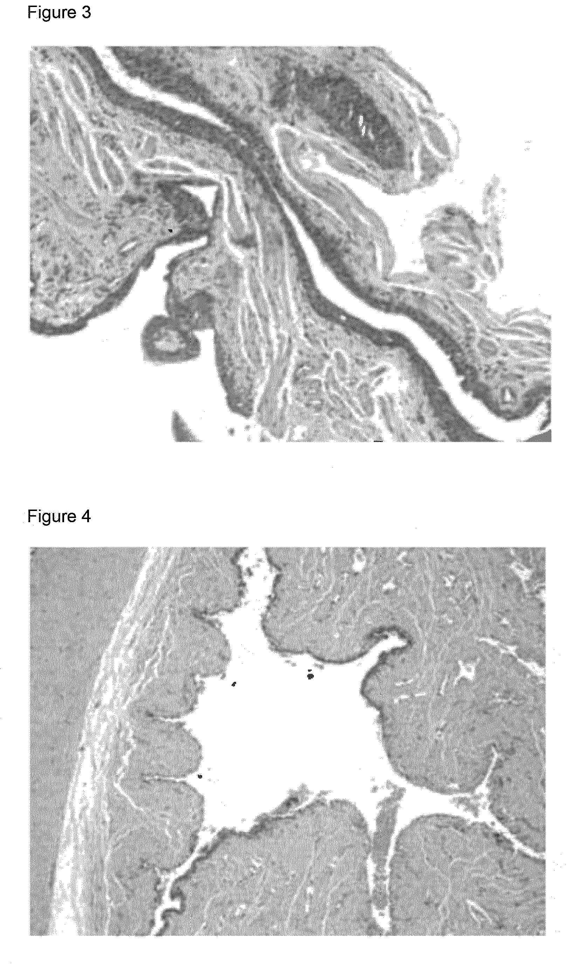

Les photographies des coupes histologiques des biopsies ainsi obtenues sont présentées en figure 2 pour la condition témoin (autogreffe) et en figure 3 pour le greffon selon l'exemple 1.

Elles démontrent des résultats similaires pour l'allogreffe ou la greffe de greffon selon l'exemple 1 présentant une forme partiellement tubulaire.

Six semaines après la greffe, les lapins greffés sont mis à mort après prémédication. De nouvelles analyses semi quantitatives selon les normes ISO 10993-6:2016 et la norme ISO 10993-10:2021 et histologiques (hématoxyline-éosine) après biopsies urétrales sont réalisées.

Pour l'analyse semi quantitative, des coupes histologiques sur trois niveaux de profondeurs séparés par 150µm (proximal ou P, médial ou M, et distal ou D) ont été produites.

Les résultats de l'analyse semi quantitative sont présentés dans la Tableau 2.

**Tableau 2**

| | | Témoin : allogreffe | | | Greffon selon l'exemple 1 | | |
|---|---|---|---|---|---|---|---|
| | Profondeur | P | M | D | P | M | D |
| Types de cellules inflammatoires | Lympocytes | 0 | 0 | 0,2 | 0 | 0 | 0 |
| | Polymorphonucléaires | 0 | 0 | 0 | 0 | 0 | 0 |
| | Cellules plasmatiques | 0 | 0 | 0,2 | 0 | 0 | 0 |
| | Macrophages | 0 | 0 | 0 | 0 | 0 | 0 |
| | Cellule géante multinucléée | 0 | 0 | 0 | 0 | 0 | 0 |
| Réponse lymphocytaire | Fibrose | 0 | 0 | 0 | 0 | 0 | 0 |
| | Neo-vascuralisation | 0 | 0 | 0 | 0 | 0 | 0 |
| | Congestion vasculaire | 0 | 0 | 0 | 0 | 0 | 0 |
| | Oedème | 0 | 0 | 0 | 0 | 0 | 0 |
| | Necrose | 0 | 0 | 0 | 0 | 0 | 0 |
| | Infiltration graisseuse | 0 | 0 | 0 | 0 | 0 | 0 |
| | Epithélium | 0,1 | 0,1 | 0,5 | 0,6 | 0,7 | 0,7 |

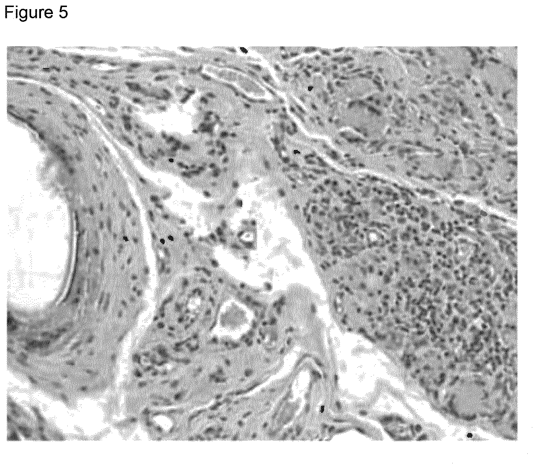

Les photographies des coupes histologiques des biopsies ainsi obtenues sont présentées en figure 4 pour la condition témoin (autogreffe) et en figure 5 pour le greffon selon l'exemple 1.

Elles démontrent des résultats similaires pour l'allogreffe ou la greffe de greffon selon l'exemple 1 présentant une forme partiellement tubulaire.

Ces résultats démontrent que le greffon selon l'invention est utilisable pour la reconstruction urétrale du fait de sa forme, son étanchéité et sa souplesse compatible avec celle de l'urètre physiologique, ainsi que sa capacité d'intégration en particulier pour la vascularisation et/ou la cicatrisation de l'urètre, et/ou la capacité à permettre la colonisation de l'épithélium urétral.

## Revendications

1. Greffon utilisable pour la reconstruction urétrale **caractérisé en ce qu'**il présente une forme partiellement tubulaire non collabable, ledit greffon comprenant au moins une veine ombilicale ou un segment de veine ombilicale disposé de telle sorte que l'épithélium vasculaire est orienté vers la face intérieure de la courbure.

2. Greffon selon la revendication 1, **caractérisé en ce que**, sur un plan transversal, la distance entre les deux points les plus éloignés de face intérieure de la courbure du greffon est comprise dans un intervalle allant de 2 à 15 mm.

3. Greffon selon l'une quelconque des revendications précédentes, **caractérisé en ce que**, sur un plan transversal, la distance entre les deux points les plus éloignés de face extérieure du greffon est comprise dans un intervalle allant de 5 à 25 mm.

4. Greffon selon l'une quelconque des revendications précédentes, **caractérisé en ce que** ledit greffon est lyophilisé.

5. Greffon selon l'une quelconque des revendications précédentes, **caractérisé en ce que** ledit greffon est apte à être utilisé dans le traitement des sténoses urétrales, et/ou des hypospadias.

6. Greffon selon l'une quelconque des revendications précédentes, **caractérisé en ce que** le segment de veine ombilicale correspond à la portion de la veine ombilicale située dans les 15 centimètres les plus proches du placenta.

7. Greffon selon l'une quelconque des revendications précédentes, **caractérisé en ce que** ledit greffon est stérile et/ou viro-inactivée.

8. Greffon selon l'une quelconque des revendications précédentes, **caractérisée en ce que** la veine ombilicale n'a pas subi de traitement par une solution d'aldéhyde.

9. Greffon selon l'une quelconque des revendications précédentes, **caractérisée en ce que** les protéines de la veine ombilicale ne sont pas réticulées.

10. Greffon selon l'une quelconque des revendications précédentes, **caractérisée en ce que** la veine ombilicale n'est pas siliconée.

11. Procédé de préparation d'un greffon selon l'une quelconque des revendications 1 à 11 comprenant les étapes de :
a) on dispose d'au moins une veine ombilicale ou un segment de celle-ci ;
b) on lyophilise ladite veine ombilicale ou le segment de celle-ci,
b₁) on procède à la découpe longitudinale et/ou transversale du greffon,
c) on obtient un greffon utilisable pour la reconstruction urétrale présentant une forme partiellement tubulaire non collabable, et comprenant au moins une veine ombilicale ou un segment de veine ombilicale disposée de telle sorte que l'épithélium vasculaire est orienté vers la face intérieure de la courbure,
ledit procédé ne comprenant pas d'étape d'aplatissement de la veine ombilicale.

12. Procédé de préparation d'un greffon selon la revendication 11 **caractérisé en ce qu'**il comprend une étape a₁) après l'étape a), de découpe de la gelée de Wharton.

13. Procédé de préparation d'un greffon selon la revendication 12, **caractérisé en ce que** l'étape a₁) de découpe de la gelée de Wharton est réalisée de telle sorte qu'il persiste une épaisseur résiduelle de gelée de Wharton d'environ 1 à 5 millimètres.

14. Procédé de préparation d'un greffon selon l'une quelconque des revendications 11 à 13, **caractérisé en ce qu'**il comprend en outre, avant l'étape b) une étape a₃) de viro-inactivation.

15. Procédé de préparation d'un greffon selon l'une quelconque des revendications 11 à 14, **caractérisé en ce qu'**il ne comprend pas d'étape de traitement par une solution aqueuse d'aldéhyde et/ou une solution organique de silicone.

16. Procédé de préparation d'un greffon selon l'une quelconque des revendications 11 à 15, **caractérisé en ce qu'**il ne comprend pas d'étape de réticulation.
